# EUROPEAN PATENT APPLICATION

(11) **EP 4 283 301 A1**
(43) Date of publication of application: **29.11.2023**
(21) Application number: 22742675.6
(22) Date of filing: 21.01.2022
(51) Int. Cl.: G01N 33/66, C12Q 1/28, C12Q 1/37, G01N 33/52

(54) **METHOD AND DEVICE FOR EVALUATING PROTEIN GLYCATION DEGREE**

(30) Priority: 23.01.2021 JP 2021009215
(71) Applicant: Provigate Inc., Tokyo 113-0033 (JP)
(72) Inventor: KATAYAMA, Norikazu, Tokyo 113-0033 (JP); KUMAGAI, Yoko, Tokyo 113-0033 (JP); ITO, Narushi, Tokyo 113-0033 (JP); MIYAUCHI, Noriko, Tokyo 113-0033 (JP)
(74) Representative: Michalski Hüttermann & Partner Patentanwälte mbB
(86) International application number: PCT/JP2022/002141
(87) International publication number: WO 2022/158556

(57) **Abstract**

An embodiment according to the present disclosure provides a method for evaluating a degree of glycation of a protein, the method including providing a solution potentially containing a target protein, bringing the solution into contact with a color indicator, bringing the solution into contact with protease, using a first reaction between the color indicator and the target protein to determine a concentration of the target protein in the solution, using a second reaction between the protease and the target protein to determine a concentration of the target protein having been glycated, and determining, based on the concentration of the target protein and the concentration of the target protein having been glycated, a degree of glycation of the target protein.

## Description

### Technical Field

The present disclosure relates to a method and an apparatus for evaluating a degree of glycation of a protein.

### Background Art

In the related art, glycated proteins are measured by high-performance liquid chromatography (HPLC) or the enzymatic method, for example. HPLC is a simple and easy measurement method, but isolation and quantification of the target component using a plurality of columns take several tens of minutes and require a plurality of high-pressure pumps, so that a large-sized apparatus is used. The enzymatic method is a commonly used, simple and easy measurement method, but the measurement of the total protein amount (protein concentration) and the measurement of the glycated protein amount (glycated protein concentration) need to be separately performed using different color reagents. In the case of using the colorimetric method, the absorption peaks of the non-glycated protein and the glycated protein are close to each other. Thus, in the case of the measurements performed at the same time, the absorption peak of one is affected by the peak of the other and accurate measurements of both are hindered. Thus, in order to measure at the same time the non-glycated protein and the glycated protein by the colorimetric method, a step of, after the measurement of one, removing the color is necessary.

Alternatively, the colorimetric method in the related art can be used to separately measure the concentration of the non-glycated protein and the concentration of the glycated protein. In order to perform this, two different measurement methods need to be combined together. However, in the two measurements, measurement errors separately occur. For example, different dilution ratios result in different errors. Specifically, in the case of calculating the degree of glycation of a protein, errors different between the denominator (protein concentration) and the numerator (glycated protein concentration) can occur. Thus, there is a risk of a large measurement error.

### Summary of Invention

Some embodiments according to the present disclosure provide a method for evaluating the degree of glycation of a protein. The method includes providing a solution potentially containing a target protein. In some embodiments, the method includes bringing a color indicator into contact with the solution and using a first reaction between the color indicator and the target protein to determine the concentration of the target protein in the solution. In some embodiments, the method includes bringing protease into contact with the solution and using a second reaction between the protease and the target protein to determine the concentration of the target protein having been glycated. In some embodiments, the method further includes determining, based on the concentration of the target protein and the concentration of the target protein having been glycated, the degree of glycation of the target protein.

In some embodiments according to the present disclosure, as an example, absorbance may be used for measuring the albumin concentration and a hydrogen peroxide electrode may be used for measuring the glycated albumin. Stated another way, different detection methods can be used for measuring them. For example, in the colorimetric method in the related art, absorbances at similar wavelengths (for example, 546 nm and 600 nm) are separately measured; by contrast, the above-described method enables measurements performed at the same time. Furthermore, for example, the dilution errors in the sample weighing and the reagent can be made equal to each other. For example, in calculation of the ratio (GA level, GA%) from the albumin concentration and the glycated albumin concentration, errors of the denominator and the numerator cancel each other out, to improve the measurement accuracy. The degree of glycation of the protein can be simply, easily, and accurately evaluated.

Additional aspects and advantages of the present disclosure will become readily apparent to those skilled in this art from the following detailed description, wherein only exemplary embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### Brief Description of Drawings

[Fig. 1] Fig. 1 is a flow chart of a method for evaluating the degree of glycation of a protein according to an embodiment.
[Fig. 2] Fig. 2 is a flow chart of a method for evaluating the degree of glycation of a protein according to an embodiment.
[Fig. 3] Fig. 3 is a graph illustrating changes in absorbance with time for a plurality of albumin concentrations in an example.
[Fig. 4] Fig. 4 is a graph illustrating a calibration curve of an albumin concentration based on absorbance in an example.
[Fig. 5] Fig. 5 is a graph illustrating the albumin concentration relation between a method according to an example and a standard method, for real samples.
[Fig. 6] Fig. 6 is a graph illustrating the influence of the presence or absence of BCP on a GA concentration measurement in an example.
[Fig. 7] Fig. 7 is a graph illustrating changes in absorbance with time for a plurality of albumin concentrations in an example.
[Fig. 8] Fig. 8 is a graph illustrating changes in the output of a sensor with time for a plurality of albumin concentrations in an example.
[Fig. 9] Fig. 9 is a graph illustrating the relation between the GA measured level determined by a method according to an example and the GA level determined by a standard method.
[Fig. 10] Fig. 10 is a graph illustrating changes in absorbance with time for a plurality of albumin concentrations in an example.
[Fig. 11] Fig. 11 is a graph illustrating the relation between the GA level determined by a method according to an example and the GA level determined by a standard method.
[Fig. 12] Fig. 12 is a block diagram of an apparatus configured to evaluate the degree of glycation of a protein according to an embodiment.
[Fig. 13] Fig. 13 is a block diagram of an apparatus configured to evaluate the degree of glycation of a protein according to an embodiment.
[Fig. 14] Fig. 14 is a block diagram of an apparatus configured to evaluate the degree of glycation of a protein according to an embodiment.
[Fig. 15] Fig. 15 is a block diagram of an apparatus configured to evaluate the degree of glycation of a protein according to an embodiment.
[Fig. 16] Fig. 16 is a graph of absorbance illustrating the influence of the presence or absence of SDS on an improved BCP method in an example.

### Description of Embodiments

### <Measurement Target>

In some embodiments, a subject (target) may include a human being or may be a human being. In some embodiments, the subject may include an animal other than human beings or may be an animal other than human beings. The subject may include a mammal or may be a mammal. The subject may be, for example, but not limited to, a working animal, a domestic animal, a pet, or a wild animal.

A sample to be measured may be a solution. The "solution" may be a body fluid, a solution derived from a body fluid, or a dilute fluid of a body fluid. The solution may be a solution that is not a body fluid (derived from a non-body fluid), or may be a mixture of a body fluid or a solution derived from a body fluid and a solution derived from a non-body fluid. The solution may be a solution used for a sample measurement or a solution used for a calibration measurement. For example, the solution may be a standard solution or a calibration solution. For example, the solution may be a liquid intentionally or deliberately free from a target substance to be measured, as it is used for calibration or the like. The sample to be measured may be a specimen. The solution may be a solution containing a chemical substance.

The "body fluid" may be lymph fluid, may be tissue fluid, such as interstitial fluid, intercellular fluid, or interstitial fluid, body cavity fluid, serosal cavity fluid, pleural fluid, ascites fluid, pericardial fluid, cerebrospinal fluid (spinal fluid), joint fluid (synovial fluid), and aqueous humor of the eye (aqueous). The body fluid may be a digestive juice, such as saliva, gastric juice, bile, pancreatic juice, or intestinal juice, and may be sweat, tears, nasal mucus, urine, semen, vaginal fluid, amniotic fluid, or milk. The body fluid may be a body fluid of an animal or may be a body fluid of a human being. The "body fluid" may be a solution. The solution contains a target substance to be measured. The solution may contain a physiological buffer, such as phosphate-buffered saline (PBS) or N-tris(hydroxymethyl)methyl-2-aminoethanesulfonic acid buffer solution (TES), containing a substance to be measured. The solution is not particularly limited as long as it contains the substance to be measured.

The solution may contain a target substance to be measured. The solution may have the possibility to contain the target substance to be measured. In some embodiments, the target substance to be measured may be, for example, a molecule, an ion, a macromolecule, or a biomolecule. The target substance to be measured may contain a biomolecule. The target substance to be measured may be a protein or a glycated protein, for example. For example, the solution may be tears, and the target substance to be measured may be albumin, glycoalbumin, hemoglobin, and/or glycohemoglobin contained in tears. Alternatively, the target substance to be measured may be albumin, glycoalbumin, hemoglobin, or glycohemoglobin in blood, serum, or plasma, or may be albumin, glycoalbumin, hemoglobin, or glycohemoglobin in interstitial fluid, urine, or saliva. The albumin may be oxidized albumin (HNA) or reduced albumin (HMA). In some embodiments, the target substance to be measured may be an advanced glycation end product (AGE). In some embodiments, the target substance to be measured may be a glycated lipid.

### <Sensor>

A sensor may be, for example, a chemical sensor, a biosensor, or an ion sensor (hereinafter sometimes referred to as a "sensor", a "biochemical sensor", a "chemical sensor", or an "electrochemical sensor"). The sensor may include multiple sensors.

The sensor may include an electrode. The electrode may be an amperometric electrode. The electrode may include a hydrogen peroxide electrode. The electrode may include an oxygen electrode. The electrode may be a potentiometric electrode. The electrode may be an electrode for ion detection (for example, a pH electrode, a cyanide ion electrode, or an iodide ion electrode).

In some embodiments, the sensor may output an electrical signal. In some embodiments, the sensor may output a current signal. The sensor may output a voltage signal or an electric charge. The sensor may be electrically coupled to, for example, an ammeter or a voltmeter.

### <Measurement of Hydrogen Peroxide>

In some embodiments, the produced glycated amino acid may be reacted with ketoamine oxidase to generate hydrogen peroxide. In some embodiments, the amount (concentration) of hydrogen peroxide generated may be measured. In some embodiments, the amount (concentration) of hydrogen peroxide may be measured electrically.

The term "ketoamine oxidase" generally refers to an oxidase that recognizes a ketoamine structure of a glycated amino acid or a peptide or peptide fragment containing a glycated amino acid residue and oxidizes the glycated amino acid to produce an amino acid, glucosone (a-ketoaldehyde), and hydrogen peroxide. Thus, ketoamine oxidase generates hydrogen peroxide in a concentration proportional to or related to the concentration of the peptide or peptide fragment containing the glycated amino acid or glycated amino acid residue it recognizes.

The ketoamine oxidase may be a dehydrogenase, a kinase, or an oxidase. The ketoamine oxidase may be fructosyl amino acid oxidase (FAOD), fructosyl peptide oxidase, fructosyl valyl histidine oxidase, fructosyl amine oxidase, amadoriase, fructosyl amine dehydratase, or a modified form thereof.

In some embodiments, the ketoamine oxidase may be an oxidase that acts on an amino acid or peptide in which an α-amino group is glycated (Group I ketoamine oxidase). The amino acid or peptide may be valine, glycine, valyl histidine. A glycated hemoglobin sensor or glycated hemoglobin A1c (HbA1c) sensor can be configured by using an oxidase that acts on an amino acid or a peptide having a glycated α-amino group.

In some embodiments, the ketoamine oxidase may be an oxidase that acts on an amino acid or peptide having a glycated ε-amino group (Group II ketoamine oxidase). The amino acid may be lysine. The glycated amino acid may be fructosyl lysine. A glycoalbumin sensor can be configured by using an oxidase that selectively acts on an amino acid or peptide having a glycated ε-amino group.

In some embodiments, the ketoamine oxidase may be an oxidase that acts on an amino acid or peptide having a glycated α-amino group and/or a glycated ε-amino group (Group III ketoamine oxidase). The amino acid or peptide may be lysine, valine, glycine, or valyl histidine. A glycated protein sensor can be configured by using an oxidase that acts on an amino acid or a peptide having a glycated α-amino group and/or a glycated ε-amino group.

In some embodiments, the sensor may include a detector. The detector may be a hydrogen peroxide detector. The "hydrogen peroxide detector" (hydrogen peroxide sensor) may be an electrochemical electrode or a hydrogen peroxide electrode. The hydrogen peroxide electrode may include a counter electrode, a reference electrode, and a working electrode. In an embodiment, the detector may detect oxygen. For example, the amount or concentration of oxygen that decreases because of an enzyme reaction may be detected. Oxygen detection is considered to be relatively insensitive to molecules and ions that are noise sources and resistant to interference. Oxygen consumption may be measured by oxygen detection. The detector is saturated with air and may be used for enzyme sensing. The detector may be configured to perform multiple detection methods selectively or in combination.

### <Noise Reduction Using Ion Exchange Resin>

In some embodiments, the sensor may include an ion-exchange resin on the detector. The sensor may include an ion-exchange resin between a ketoamine oxidase layer and a hydrogen peroxide electrode.

For example, a cation-exchange resin, such as Nafion (registered trademark), can be used to inhibit or prevent, for example, ascorbic acid, uric acid, and in particular, anions and so forth present in the body fluid from permeating and reaching the detector. For example, an anion-exchange resin, such as polypyrrole, can be used to inhibit or prevent dopamine, in particular, cations and so forth, from passing through and reaching the detector.

The ion-exchange resin may contain one, a plurality of, or at least one type of ion-exchange resin. The ion-exchange resin may be configured to have one, more than one, or at least one type of layer.

### <Molecularly Imprinted Polymer Sensor>

The sensor may include a molecular imprinted polymer (MIP) membrane. A change in charge or potential caused by the molecular recognition of MIP may be detected (potentiometric type). A change in current (e.g. mediator current) flowing through the electrode caused by the molecular recognition of MIP may be detected (amperometric type).

### <Protease>

"Protease" is a generic term for peptide-bond hydrolases that hydrolyze and catabolize proteins and polypeptides. A protease may be an enzyme that breaks down a protein into peptide fragments. When a protein contains a glycated amino acid residue, peptide fragments produced by the action of a protease may include peptide fragments containing a glycated amino acid residue and peptide fragments that are not glycated at all.

The "protease" may be a protease derived from an animal, a protease derived from a plant, or a protease derived from a microorganism. The protease may be an exopeptidase or an endopeptidase. The protease may be an aspartic protease, a metalloprotease, a serine protease, or a thiol protease.

The "protease" may include multiple types or kinds of protease, and may include one type or kind of protease. For example, the protease may include one or both of an endopeptidase and an exopeptidase. The degradation efficiency may be increased by mixing multiple proteases.

The animal-derived protease may be, for example, trypsin, chymotrypsin, elastase, bovine pancreatic protease, cathepsin, calpain, protease type-I, protease type-XX, aminopeptidase N, carboxypeptidase, or pancreatin (a mixture of multiple enzymes such as protease and amylase).

The plant-derived protease may be, for example, papain, bromelain, gingipain, kallikrein, ficin, or chymopapain.

The protease derived from a microorganism may be, for example, a Bacillus-derived protease, an Aspergillus-derived protease, a Penicillium-derived protease, a Streptomyces-derived protease, a Lysobacter-derived protease, a yeast-derived protease, a Tritirachium-derived protease, a Thermus-derived protease, a Pseudomonas-derived protease, or an Achromobactaer-derived protease.

In some embodiments, the protease may be provided in a liquid form and mixed with a solution. In some embodiments, the protease may be added to or mixed with a solution in solid form (for example, a powder). In some embodiments, the protease may be loaded on a support material and introduced into a solution. The protease may be supported on, for example, a flat plate, a curved surface, a sphere, a bead, a biomolecule such as a protein, or a polymer.

### <Optical Measurement>

The term "optical measurement" used herein generally refers to determining optical properties of a substance using an optical element or device. In some embodiments, optical measurement of the substance of interest may be measured. In some embodiments, the properties of a substance bound to or associated with a target substance (hereinafter, a substance (for example, a reagent) chemically, biologically, or physically bound to or associated with the target substance, even if it is not the target substance itself) may be measured. Properties of the reagent may be measured. The reagent may be referred to as a "target substance".

In some embodiments, the optical measurement may include a spectroscopic measurement. For example, the absorbance of the target substance may be measured. In some embodiments, a color indicator corresponding to the target substance may be introduced. The color indicator may be detected or measured.

### <Color Indicator>

The term "color indicator" used herein generally refers to a halochromic chemical compound. The color change may generally be reversible. Examples of the color indicator include, but are not limited to, acid-base indicators (pH indicators), oxidation-reduction indicators, adsorption indicators, and TLC color indicators. It changes the color of the solution in accordance with the pH. Examples of the pH indicator include, but are not limited to, bromocresol purple (BCP) and bromocresol green (BCG).

In some embodiments, BCP or BCG can be used for albumin. For example, albumin binds to bromocresol green (BCG) to form an albumin-bromocresol green complex. At about pH 4, the complex is blue in color. Thus, the albumin concentration can be determined by measuring the absorbance (BCG method). A surfactant may be added to the measurement reagent. A surfactant need not be added to the measurement reagent. For example, a protein denaturing agent (such as SDS) need not be incorporated.

For example, albumin binds with bromocresol purple (BCP) to form an albumin-bromocresol purple complex. This complex is blue in color. Thus, the albumin concentration can be determined by measuring the absorbance (BCP method). Typically, the concentration of albumin can also be measured by converting all reduced albumin into oxidized albumin by the action of an oxidizing agent and then performing a color reaction with BCP (improved BCP method). A surfactant may be added to the measurement reagent. For example, a protein denaturing agent (such as SDS) need not be incorporated.

In some embodiments, a reaction in which albumin binds with bromocresol purple (BCP) or bromocresol green (BCG) to form a complex may be referred to as a "first reaction."

In some embodiments, the color indicator may be provided in a liquid form or mixed with a solution. In some embodiments, the color indicator may be added to or mixed with a solution in a solid form (for example, a powder).

### <Measurement of Absorbance>

In some embodiments, an absorptiometer (colorimeter) may be used. The absorptiometer includes a light source and a photodetector. All or a portion of light from the light source enters a target solution. All or a portion of the light passing through the solution enters the photodetector. The light absorbed by the target substance can be analyzed.

The wavelength of the light source may be appropriately selected in accordance with a color indicator used. The absorbance may be measured, for example, at a wavelength, such as a dominant wavelength, of 600 to 630 nm for BCP and 565 to 660 nm for BCG.

In some embodiments, a change in the absorbance of the color indicator over time may be measured. In some embodiments, the absorbance may be measured at least two (or multiple) time points. For example, the concentration of protein at the beginning (before the initiation of protease degradation, t = 0) may be determined based on the measurement values at two time points. In some embodiments, the time point of initiation of protease degradation may be measured, and the concentration of protein may be determined based on measurements as a function of time from this initiation time. In some embodiments, the concentration of protein may be determined based on measurements at three or more time points. From at least three or more measured values, the concentration of original protein can be determined in some cases even when the time from the time point of initiation of protease degradation is not clear.

In some embodiments, the concentration of the protein may be determined by referring to a calibration curve, prepared in advance, presenting the relationship between the absorbance and the protein concentration. For example, a calibration curve presenting the relationship between the absorbance A (t) that changes with time (t) and its change with time (ΔA(t)/Δt) is prepared in advance. In the actual measurement, the absorbance A(t1) and its slope (ΔA(t1)/Δt) at a certain time point (t = t1) are determined. From this, the absorbance A (t = t0) at the degradation start time t = t0 of the protease can be determined.

In some embodiments, the measurement of the glycated protein may be started after a predetermined time has elapsed from the time point of the initiation of the protease degradation. In some embodiments, the measurement of the glycated protein may be started when the absorbance A is sufficiently small. In some embodiments, when the change in absorbance with time (ΔA/Δt) is substantially zero, it is determined that sufficient degradation by the protease has been completed, and measurement of the glycated protein may be started, or the measurement result of the glycated protein at that time may be used. In some embodiments, when the change in absorbance over time reaches a predetermined value, it may be determined that degradation by a predetermined protease has been achieved, and measurement of the glycated protein may be started, or the measurement result of the glycated protein at that time may be used.

In some embodiments, calibration of absorbance measurements may be performed. Optical components including an optical measurement cell, a light-emitting element, a light-receiving element, and so forth may be calibrated. For example, the absorbance may be measured before (for example, when the optical measurement cell is empty, in its initial state, etc.) and after (for example, when the measurement solution has been introduced into the optical measurement cell) the introduction of the solution into the optical components. A difference between the absorbances may be determined.

### <Steps of Measurement>

Some embodiments according to the present disclosure provide a method for measuring a protein, the method including providing a solution potentially containing a target protein, adding a color indicator to this solution, and subsequently or simultaneously adding protease. This method can be used for measuring a glycated protein. However, applications of this method are not limited to this. This method can be used for various other methods.

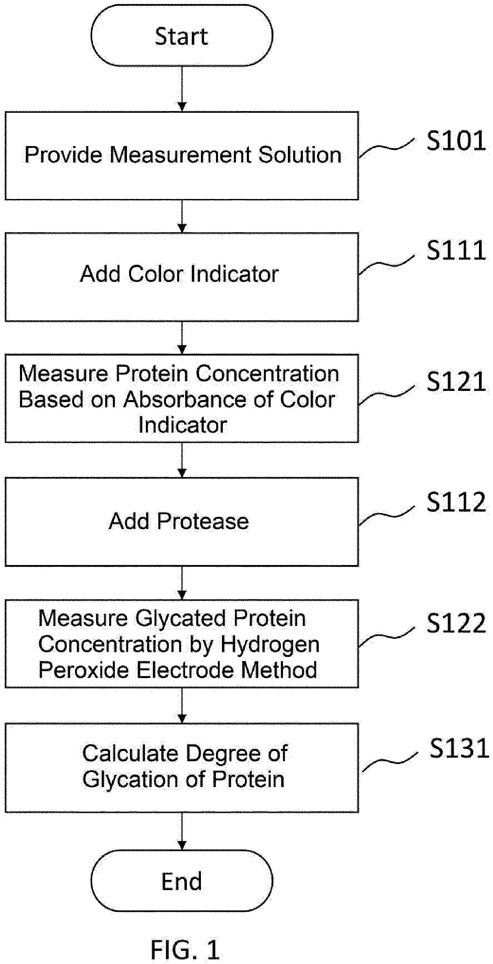

Fig. 1 illustrates a flow chart of a method for evaluating the degree of glycation of albumin according to an embodiment.

In Step S101, a solution potentially containing albumin as a protein serving as the target to be measured is provided.

In Step S111, as a color indicator, BCP is added.

In Step S121, the absorbance measurement of BCP is performed. From the absorbance, the albumin concentration is determined.

In Step S112, to the measurement solution to which BCP has been added, protease is added. The protease begins to degrade albumin into peptides or amino acids.

In Step S122, a ketoamine oxidase that specifically reacts with glycated amino acids among the peptides or amino acids generated by the digestion reaction caused by the protease is caused to act to thereby generate hydrogen peroxide. The hydrogen peroxide is measured using an electrode. In this way, the concentration of glycated albumin (GA) in the solution is determined.

In Step S131, the ratio of the determined glycated albumin (GA) to the albumin concentration is determined as the GA level.

In Fig. 1, the color indicator is added (Sill), the absorbance is measured (S121), and subsequently the protease is added (S112). In some embodiments, before the addition of protease, the absorbance measurement may be completed. In some embodiments, before completion of the absorbance measurement or during the absorbance measurement, protease may be added.

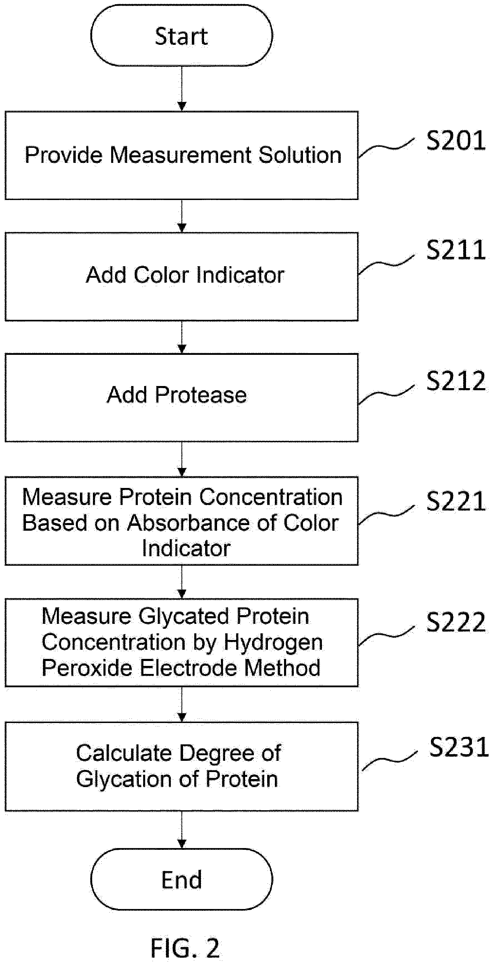

Fig. 2 illustrates a flow chart of a method for evaluating the degree of glycation of albumin according to an embodiment.

In Step S201, a solution potentially containing albumin as a protein serving as the target to be measured is provided.

In Step S211, as a color indicator, BCP is added.

In Step S212, to the measurement solution to which BCP has been added, protease is added. The protease begins to degrade albumin into amino acids.

In Step S221, the absorbance measurement of BCP is performed. From the absorbance, the albumin concentration is determined. Since degradation of albumin proceeds, the absorbance decreases with time. For example, at the time point of introduction of protease or the time point of initiation of degradation and at a time point after a lapse of a predetermined time from the time point of the initiation, the absorbances can be measured, so that the concentration of albumin initially contained in the measurement solution can be determined.

In Step S222, a ketoamine oxidase that specifically reacts with glycated amino acids among the peptides or amino acids generated by the digestion reaction caused by the protease is caused to act to thereby generate hydrogen peroxide. The hydrogen peroxide is measured using an electrode. In this way, the concentration of glycated albumin (GA) in the solution is determined.

In Step S231, the ratio of the determined glycated albumin (GA) to the albumin concentration is determined as the GA level.

In Fig. 2, the color indicator is added (S211) and subsequently the protease is added (S212). However, the order of Steps is not limited to this. In some embodiments, the addition of the color indicator (S211) and the addition of the protease (S212) may be performed substantially at the same time. For example, the protease and the color indicator may be mixed together in advance and the resultant mixture solution may be added to the measurement solution.

In Fig. 2, the albumin concentration is measured (S221) and subsequently the glycated albumin concentration is measured (S222). However, the order of Steps is not limited to this. In some embodiments, the measurement of the albumin concentration (S221) and the measurement of the glycated albumin concentration (S222) may be performed substantially at the same time. In some embodiments, the color indicator is added (S211), the protease is added (S212), and subsequently the solution to which both of them have been added may be split. In one of the split solutions, the albumin concentration may be measured while, in the other, the glycated albumin concentration may be measured. In some embodiments, unlike Fig. 2, the protease and the color indicator are not necessarily added separately. For example, the protease and the color indicator may be mixed together in advance and the resultant mixture solution may be added to the measurement solution.

The color indicator is brought into contact with the solution potentially containing the target protein and subsequently the first reaction between the color indicator and the target protein is used to determine the concentration of the target protein in the solution. Similarly, the protease is brought into contact with the solution potentially containing the target protein and subsequently the second reaction between the protease and the target protein is used to determine the concentration of the glycated target protein (also referred to as glycated target protein concentration, glycated protein concentration).

In some embodiments, the second reaction may include causing the generated glycated amino acids to react with ketoamine oxidase to generate hydrogen peroxide.

Except for the above-described order, the order of four items of the introduction of the color indicator, the measurement of the first reaction between the color indicator and the target protein, the introduction of the protease, and the measurement of the second reaction between the protease and the target protein is not limited.

In some embodiments, except for the orders in which the measurement solution and the indicator are mixed together and subsequently the indicator mixture solution is measured in terms of protein, and the measurement solution and the protease are mixed together and subsequently the protease mixture solution is measured in terms of glycated protein, the orders of steps are not limited.

In some embodiments, after the measurement solution and the indicator are mixed together, the indicator mixture solution may be mixed with the protease. In some embodiments, the measurement solution and the indicator may be mixed together, the indicator mixture solution may be measured in terms of protein, the indicator mixture solution may be mixed with the protease, and the indicator-protease mixture solution (mixture solution of the indicator and the protease) may be measured in terms of glycated protein. In some embodiments, the measurement solution and the indicator may be mixed together, the indicator mixture solution may be mixed with the protease, the indicator-protease mixture solution (mixture solution of the indicator and the protease) may be measured in terms of glycated protein, and the protease mixture solution may be measured in terms of protein. In some embodiments, mixing together of the measurement solution and the indicator, mixing the indicator mixture solution with the protease, measuring the indicator-protease mixture solution (mixture solution of the indicator and the protease) in terms of glycated protein, and measuring the indicator-protease mixture solution in terms of protein may be performed in this order.

In some embodiments, after mixing together of the measurement solution and the protease, the protease mixture solution may be mixed with the indicator. In some embodiments, mixing together of the measurement solution and the protease, measuring the protease mixture solution in terms of glycated protein, mixing the protease mixture solution with the indicator, and measuring the protease-indicator mixture solution in terms of protein may be performed in this order. In some embodiments, mixing together of the measurement solution and the protease, mixing the protease mixture solution with the indicator, measuring the indicator-protease mixture solution in terms of glycated protein, and measuring the indicator-protease mixture solution in terms of protein may be performed in this order. In some embodiments, mixing together the measurement solution and the protease, mixing the protease mixture solution with the indicator, measuring the indicator-protease mixture solution in terms of protein, and measuring the indicator-protease mixture solution in terms of glycated protein may be performed in this order.

In some embodiments, the measurement solution may be mixed with the indicator and the protease substantially at the same time.

<Calculation of Degree of Glycation>

In some embodiments, for the target protein, on the basis of the target protein concentration and the glycated protein concentration, the degree of glycation of the target protein may be determined. The degree of glycation of the protein may be determined by dividing the glycated protein concentration by the protein concentration. Such measured amounts are not necessarily limited to concentrations. The measured amounts may be absolute amounts, may be relative amounts, may be concentrations, or may be other amounts correlating to mass or concentration.

### <Others>

In some embodiments, after addition of the color indicator and addition of the protease, the protein concentration and the glycated protein concentration can be measured. In this case, the color indicator and/or the protease is added in the form of liquid and the volume is changed from that of the initial solution. However, variations due to error in the amount of the color indicator and/or the protease added occur in the same ratio for both of the protein concentration and the glycated protein concentration. Thus, they substantially do not affect the calculation of the degree of glycation. Therefore, the relatively accurate, repeatable measurement can be performed.

In some embodiments, after addition of the color indicator, the measurement of the protein concentration, addition of a solid protease, and the measurement of the glycated protein concentration can be performed. Also in this case, as with above, variations due to error in the amount of the color indicator and/or the protease added occur in the same ratio for both of the protein concentration and the glycated protein. Thus, they substantially do not affect the calculation of the degree of glycation. Therefore, the relatively accurate, repeatable measurement can be performed.

### <Conditions of Color Indicator and Protease>

In some embodiments, various conditions (such as pH and temperature) in the reaction or measurement using the color indicator and the reaction or measurement using the protease may be adjusted or changed.

In some embodiments, pH may be adjusted. Different pHs may be employed for the addition or reaction of the color indicator (first reaction) and for degradation using the protease (second reaction). For example, during addition of BCP, the pH may be adjusted to a range including an ordinarily suitable range and may be adjusted to a range of pH = 4.5 to 8.5 or pH = 5.0 to 6.8, for example. After introduction of the protease, the pH may be adjusted to a range including an ordinarily suitable range and may be adjusted to a range of pH = 6.0 to 9.0 or pH = 7.0 to 8.5, for example.

In some embodiments, the first reaction and the second reaction may be performed at substantially the same pH. For example, for both of the first reaction and the second reaction, the pH may be adjusted to a range of pH = 5.0 to 9.0 or pH = 7.0 to 8.5, for example. In some embodiments, the pH value may be different from the ordinarily suitable range. For example, the first reaction may be performed at an alkaline pH (pH = 7.0 to 9.0) while the second reaction may be performed at an alkaline pH (pH = 7.0 to 9.0). In some embodiments, after the first reaction, the second reaction may be performed.

In some embodiments, during the first reaction and the second reaction that are performed (at least partially) at the same time, a single pH is applied to both of the reactions. This value may be adjusted to a range suitable for both of them. For example, for both of the first reaction and the second reaction, the pH may be adjusted to a range of pH = 5.0 to 9.0 or pH = 7.0 to 8.5, for example. In some embodiments, the pH value may be different from the ordinarily suitable range.

In some embodiments, the temperature may be adjusted. Different temperatures may be employed for the addition or reaction of the color indicator (first reaction) and for degradation using the protease (second reaction). For example, during addition of BCP, the temperature may be adjusted to the ordinarily suitable temperature of 20°C to 70°C and, after introduction of the protease, the temperature may be adjusted to 25°C to 70°C, 50 to 70°C, or 55 to 65°C. After the absorbance measurement and before introduction of the protease, the temperature may be temporarily increased to the temperature for protease degradation. After the protease degradation, during the ketoamine oxidase reaction, the temperature may be adjusted to 25°C to 75°C, 30°C to 65°C, 35°C to 65°C, about 37°C, or about 55°C.

In some embodiments, during the first reaction and the second reaction that are performed (at least partially) at the same time, a single temperature is applied to both of the reactions. This value may be adjusted to a range suitable for both of them. For example, for both of the first reaction and the second reaction, the temperature may be adjusted to a range of 25°C to 50°C, 25°C to 45°C, or 30°C to 45°C. For example, for both of the first reaction and the second reaction, the temperature may be adjusted to a range of 25°C to 70°C, 30°C to 70°C, 40°C to 70°C, 50°C to 70°C, or 55°C to 65°C.

### <Example 1: Measurement of ALB Concentration>

In an example, an absorbance method was used to measure albumin concentration. For albumin sample solutions, a commercially available glycated albumin measurement kit (LUCICA (registered trademark) GA-L) calibrator (GA-L calibrator, ASAHI KASEI PHARMA CORPORATION) was used to prepare PBS dilution series (7.93 mg/mL, 3.97 mg/mL, and 1.98 mg/mL). As a blank sample, a solution composed of PBS alone (blank PBS) was also prepared. For a protease solution, 320 mg of Orientase 22BF (HBI Enzymes Inc.) was dissolved in 4 mL of a HEPES buffer solution. For the color reaction, a BCP solution (0.02 mM, pH: 8.0) was used. The absorbance was measured using an automated analyzer for diabetes mellitus tests DM-JACK Ex (Hitachi Chemical Diagnostics Systems Co., Ltd.) over time at intervals of 12 seconds. At time point t = 0 min, to 18 µL of such a sample solution, 180 µL of the BCP solution was added. At t = about 4 min, 18 µL of the protease solution was added. From t = 0 min to about 9 min, the absorbance was measured.

### <Generation of ALB Calibration Curve>

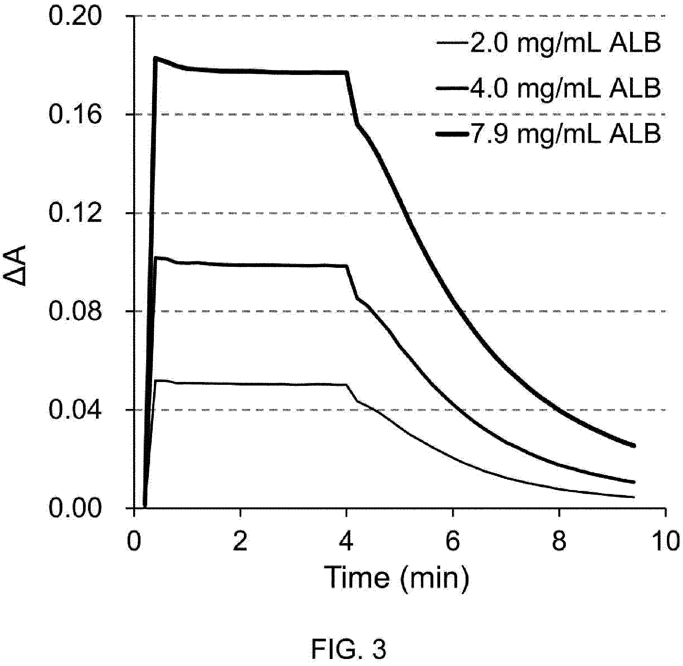

Fig. 3 illustrates changes in ΔA with time determined by subtracting, from absorbances A for the plurality of albumin concentrations, absorbance A0 of the blank PBS. Before t = 0 min, BCP was not present and hence absorbance ΔA = 0. Relative to the absorbance (ΔA = 0) at t = 0 min, after addition of BCP, changes in absorbance (ΔA) were observed. The amounts of changes in absorbance (ΔA) until t = 4 min were substantially constant. This demonstrates that the absorbance of BCP can be measured with stability.

ΔA being constant corresponds to the albumin concentration. In some embodiments, on the basis of the absorbance of a solution to which protease is not added, the albumin concentration may be determined. In Fig. 3, ALB = 1.98 mg/mL, 3.97 mg/mL, and 7.93 mg/mL respectively corresponded to ΔA = 0.05, 0.1, and 0.18 and the relation therebetween was substantially linear.

At t = 4 min, the protease was added and, as a result, the absorbances ΔA decayed. This shows that the protease degrades albumin, so that the concentration of albumin that reacts with BCP decreases. All the decay curves were similar to each other. For all of them, at any point, the ratio among the three concentrations was constant.

In some embodiments, on the basis of an absorbance value (A or ΔA) at a predetermined time from addition of protease, the albumin concentration may be determined. For example, the end point method may be used. In some embodiments, on the basis of the conversion rate of absorbance with time (dA/dt or d(ΔA)/dt), the albumin concentration may be determined. For example, rate assay may be used. In some embodiments, on the basis of the high-order differential coefficient of the absorbance (such as d2A/dt2 or d2(ΔA)/dt2), the albumin concentration may be determined.

In some embodiments, on the basis of absorbance (ΔA) at a time point after a lapse of sufficient time, the albumin concentration may be determined. In some embodiments, GA may be measured after a time point when ΔA no longer changes. For example, after t = 10 min (not shown), ΔA becomes a constant value and the degradation reaction of albumin is regarded as being complete. In some embodiments, GA may be measured at a time point after a lapse of a predetermined time (for example, after t = 10 min).

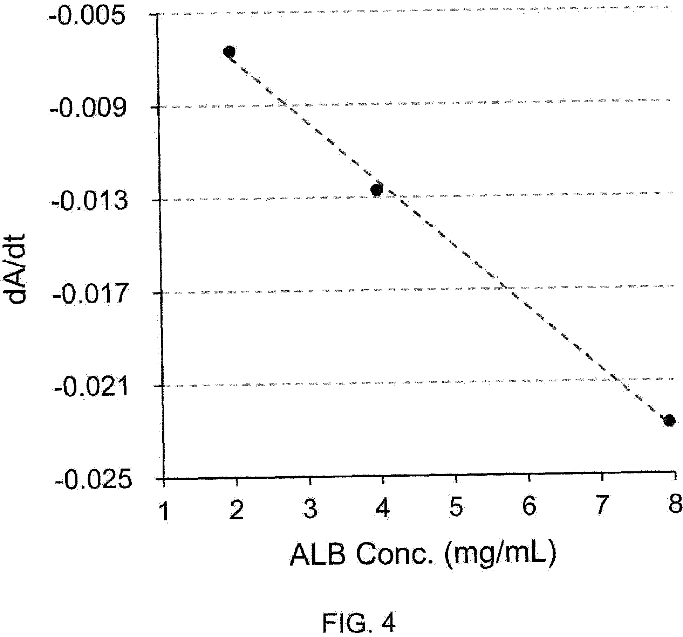

Fig. 4 illustrates a calibration curve determined on the basis of the relation between the albumin concentration and dA/dt of the sample solution. This showed such a very strongly negative correlation.

### <ALB Measurement for Real Samples>

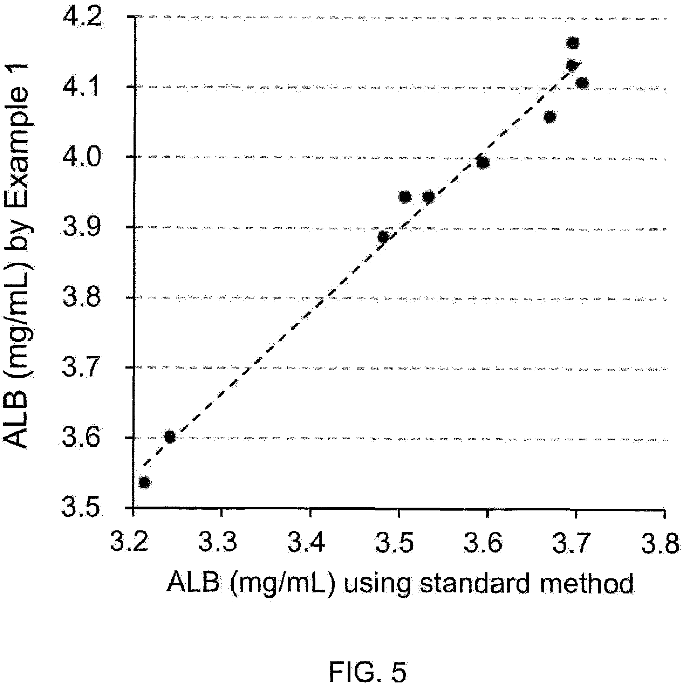

The same method as above was performed to measure the albumin concentrations of real samples of 10 healthy people. As the real samples, solutions prepared by sampling blood plasma from healthy people and diluting the blood plasma 10-fold with PBS were used. The samples were measured, using a commercially available glycated albumin measurement kit, LUCICA (registered trademark) GA-L, in terms of albumin concentration and comparison was performed. Fig. 5 illustrates the relation between both of them. This showed a very strongly positive correlation.

### <Example 2: Measurement of Glycated Albumin Concentration>

In an example, an enzyme electrode method was used to measure glycated albumin concentration.

For sample solutions, to HSA (FUJIFILM Wako Pure Chemical Corporation), glucose was added and incubated at 37°C, to prepare GA solutions (40 mg/mL) having different glycation ratios. The glycation ratio of each of the samples was determined using a commercially available glycated albumin measurement kit (LUCICA (registered trademark) GA-L, ASAHI KASEI PHARMA CORPORATION). A protease solution was prepared by dissolving 2 mg of Amano K (Amano Enzyme Inc.) in 200 µL of a TES buffer solution. To the 20 µL of the protease solution, 8 µL of a BCP solution (1 mM) was added. This solution was heat-treated and stored at 4°C.

This sample was added to an electrochemical sensor including a hydrogen peroxide electrode in which a ketoamine oxidase membrane was fixed. The output current was measured to thereby determine the glycated albumin concentration.

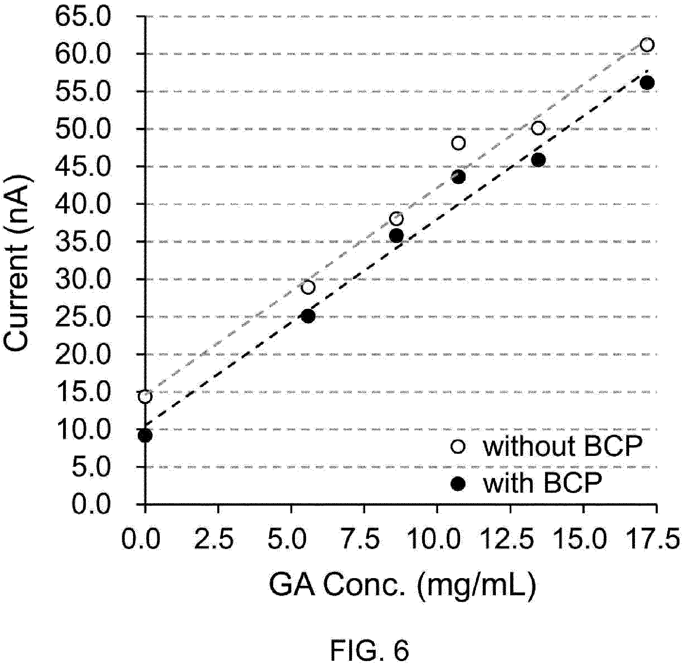

Fig. 6 illustrates the relation between glycated albumin concentration and enzyme sensor output with or without BCP. The abscissa axis indicates the glycated albumin concentration in the solution while the ordinate axis indicates the measured current value. The white circles represent the measurement "without BCP" while the black circles represent the measurement "with BCP".

Both measurements provided similar GA levels and similar concentration dependencies. This showed that the presence of BCP substantially does not affect degradation of albumin using protease or the measurement of glycated amino acids using ketoamine oxidase. Similar results were also obtained for BCG (not shown). Therefore, inferentially, addition of other color indicators substantially does not affect subsequent degradation of albumin using protease or the measurement of glycated amino acids using ketoamine oxidase.

### <Example 3: GA Level Measurement-1>

In this example, an example of the GA level measurement will be described. To a sample containing glycated albumin, BCP was added; an albumin concentration measurement based on absorbance was performed; subsequently, to the solution, protease was added; an electrochemical sensor including a hydrogen peroxide electrode in which an FAOD membrane was fixed was used to perform a glycated albumin concentration measurement. Hereinafter, this will be specifically described.

### <Generation of GA Calibration Curve>

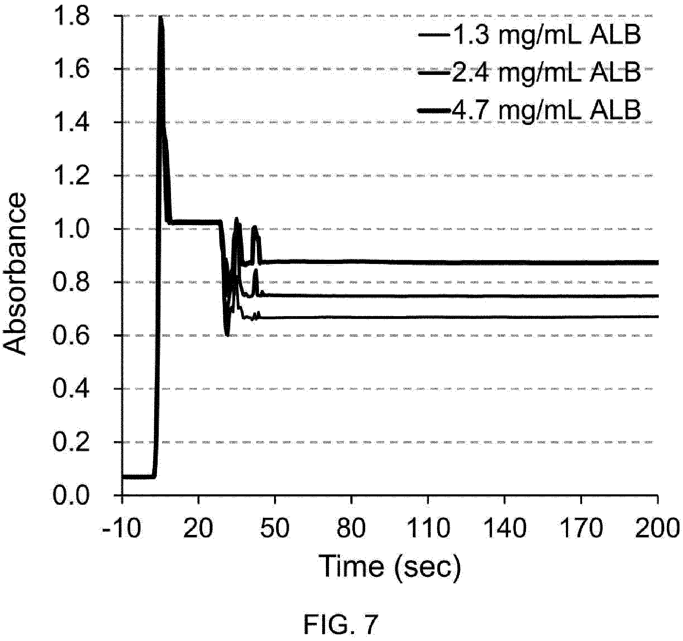

Fig. 7 illustrates an example of measurement results used for generation of a calibration curve for albumin concentration based on the absorbance measurement. For the absorbance measurement, samples containing 1.3 mg/mL, 2.4 mg/mL, or 4.7 mg/mL of albumin were used. In Fig. 7, at 0 seconds, BCP solutions (containing Sodium Dodecyl Sulfate (SDS), TritonX-100, 5,5'-Dithiobis(2-nitrobenzoic acid (DTNB), HEPES (pH: 8.0), and NaCl) were introduced into optical measurement cells. At about 30 seconds, the albumin samples were added to the BCP solutions. This resulted in decreases in the absorbances. Subsequently, pipetting was performed to mix the solutions. The spikes and variations observed until about 40 seconds were caused by movements of the solutions due to the mixing and blocking of the optical paths due to the pipette. After a lapse of about 50 seconds, the absorbances of all the albumin samples were constant. As was clear from Fig. 7, the absorbances showed albumin-concentration dependency; thus, the relation between albumin concentration and absorbance was determined and its calibration curve was generated (not shown).

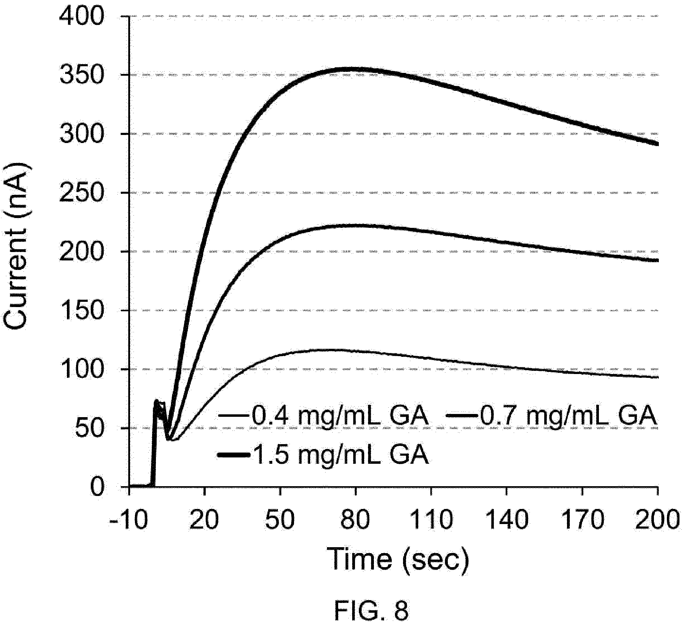

Fig. 8 illustrates an example of measurement results used for generation of a calibration curve for glycated albumin concentration in the electrochemical sensor measurement. For the measurement, samples containing 0.4 mg/mL, 0.7 mg/mL, or 1.5 mg/mL of glycated albumin were used. These solutions contained BCP. To the solutions after the absorbance measurement, protease was added and a digestion reaction was caused at 55°C for 30 minutes. Subsequently, in Fig. 8, at 0 seconds, the solutions after the digestion reaction were introduced into an electrochemical sensor. The output currents of the electrochemical sensor were measured. For all the concentrations, the values were found to reach the maximums (inflection points) in the range of about 60 seconds to about 80 seconds. In this case, for example, at 60 seconds, the relation between output current and glycated albumin concentration can be determined. Such data was used to determine the relation between glycated albumin concentration and output current of the electrochemical sensor and its calibration curve was generated.

The calibration curve of albumin concentration and the calibration curve of glycated albumin concentration determined in the above-described manner can be used to determine the GA levels of unknown samples.

### <GA Level Measurement of Unknown Samples>

Subsequently, the same method as above was used to measure GA levels in unknown samples. For measurement samples, a high GA-level sample and a low GA-level sample were mixed together in some ratios to prepare a plurality of measurement solutions. For the low GA-level sample, a human serum albumin reagent (Sigma-Aldrich) was used. The high GA-level sample was prepared by adding, to the human serum albumin reagent (Sigma-Aldrich), glucose and performing incubation. These are artificially prepared samples, but the actual GA levels are unknown.

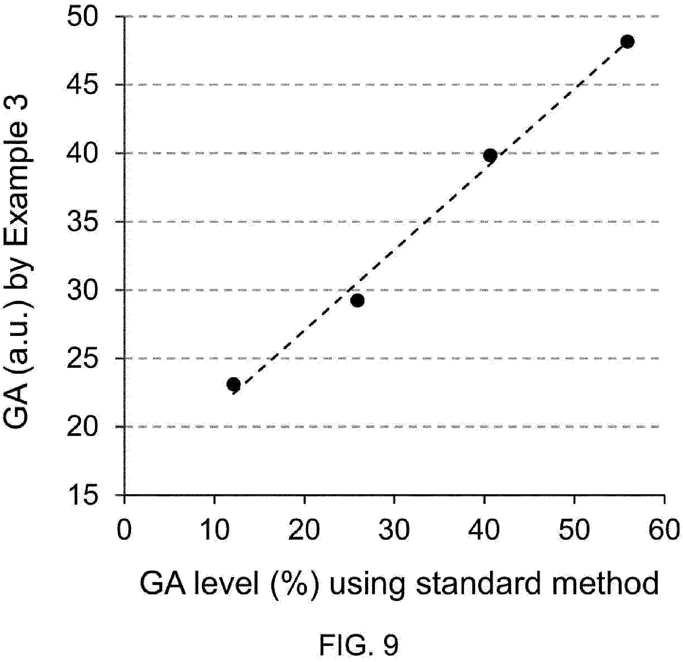

To these measurement samples, the measurement method of this example was applied, to determine the GA levels. For the same measurement samples, a standard method using a commercially available glycated albumin measurement kit (LUCICA (registered trademark) GA-L) was performed to determine GA levels (%). Fig. 9 illustrates, for the four samples having unknown different concentrations, values measured by both methods. The abscissa axis indicates GA level measured using LUCICA while the ordinate axis indicates GA level (arbitrary units, not indicating GA level (%)) provided by the measurement method according to this example. As a result, it has been demonstrated that use of one of the methods according to the present disclosure provides GA levels determined extremely well.

### <Example 4: GA Level Measurement-2>

In this example, an example of the GA level (%) measurement will be described. To samples containing glycated albumin, BCP and a protease mixture solution were added; subsequently, an optical measurement was performed to measure the albumin concentrations while an electrochemical sensor including a hydrogen peroxide electrode in which an FAOD membrane was fixed was used to measure the glycated albumin concentrations. Hereinafter, this will be specifically described.

### <Generation of ALB Calibration Curve>

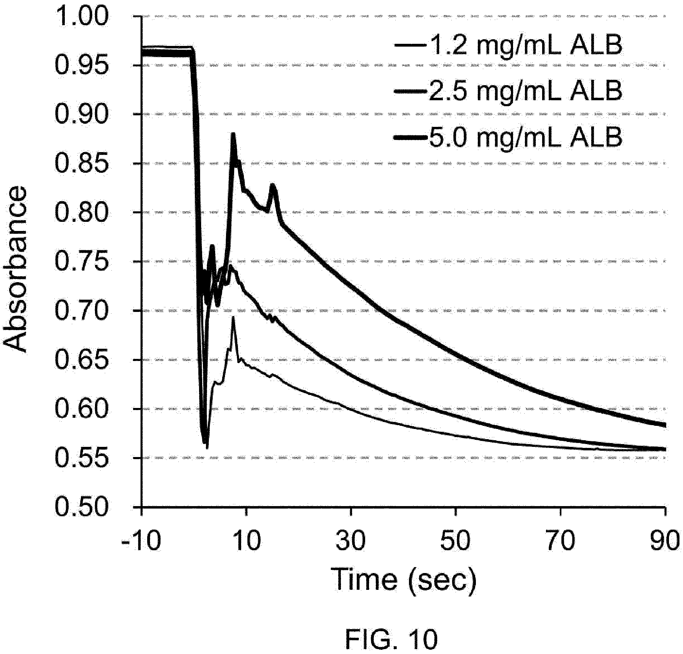

Fig. 10 illustrates an example of measurement results used for generation of a calibration curve for albumin concentration based on the absorbance measurement. For the measurement, samples containing 1.2 mg/mL, 2.5 mg/mL, or 5.0 mg/mL of albumin were used. In Fig. 10, before -10 seconds, BCP and the protease mixture solution (SDS, TritonX-100, DTNB, HEPES (pH: 8.0), NaCl, and protease were added) were introduced into optical measurement cells. At 0 seconds, the albumin samples were added to the BCP-protease mixture solutions. This resulted in decreases in the absorbances. Subsequently, pipetting was performed to mix the solutions. The spikes and variations observed until about 15 seconds were caused by movements of the solutions due to the mixing and blocking of the optical paths due to the pipette. After about 20 seconds, the absorbances decayed with stability.

Thus, absorbance-related values such as the absorbance at 20 seconds, the difference between absorbances at 20 seconds and at a time point after that (for example, at 90 seconds, and a change in absorbance with time (time derivative) are governed by the initial albumin concentration. Such data was used to determine the relation between albumin concentration and absorbance and its calibration curve was generated.

The same method as in Fig. 8 was used to determine the relation between glycated albumin concentration and the output current of the electrochemical sensor and its calibration curve was generated. (not shown)

### <GA Level (%) Measurement of Unknown Samples>

As in Example 3, measurement samples were prepared.

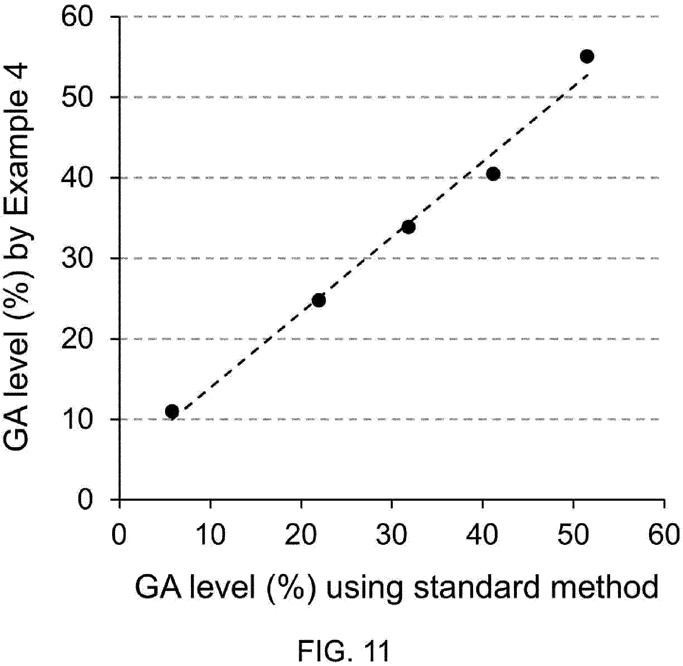

To these measurement samples, the measurement method of this example was applied, to determine the GA levels (%). For the same measurement samples, a standard method using a commercially available glycated albumin measurement kit (LUCICA (registered trademark) GA-L) was performed to determine GA levels (%). Fig. 11 illustrates, for the five samples having unknown different concentrations, the relation between values measured by both methods. The abscissa axis indicates GA level (%) provided by the standard measurement method while the ordinate axis indicates GA level (%) provided by the measurement method according to this example. As a result, it has been demonstrated that use of one of the methods according to the present disclosure provides GA levels (%) determined extremely well.

### <Example 5: Example of Weakly Alkaline Modified BCP Method>

In the above-described examples, SDS was contained; however, in other embodiments, SDS is not necessarily contained. An example in which both of the first reaction and the second reaction are performed under a weakly alkaline condition (in this example, pH = 8.0) will be described. Hereinafter, the first reaction will be described.

In general, the albumin measurement by the modified BCP method is performed using an oxidizing agent and SDS under acidic conditions. Denaturing agents for proteins, such as SDS, are considered to facilitate oxidation of albumin during introduction of an oxidizing agent such as DTNB. In this state, BCP is introduced.

On the other hand, in general, the protease reaction serving as the second reaction requires a weakly alkaline condition. In order to perform the first reaction and the second reaction at the same time, continuously, or serially, the first reaction is also preferably performed similarly under an alkaline environment. This eliminates the necessity of changing the pH.

At high pH (weakly alkaline, for example, pH 8.0), the oxidation rate is sufficiently high for the ordinary measurement efficiency and hence albumin can be sufficiently oxidized without SDS inferentially. In addition, without SDS, deformation of high-order structures of proteins can be avoided and, for example, decrease in the activity of protease can be avoided inferentially. In some embodiments, in the modified BCP method performed in a weakly alkaline condition, the solution preferably does not contain SDS.

Thus, in this example, whether the modified BCP method functions at pH = 8.0 was examined. First, GA-L calibrator (ASAHI KASEI PHARMA CORPORATION) was diluted to prepare samples having albumin concentrations of 0 g/dL, 0.23 g/dL, and 0.45 g/dL. As a weakly acidic buffer solution, a succinic acid solution (pH: 5.4) was used while, as weakly alkaline buffer solutions, an HEPES solution (pH: 8.0) and a Tricine solution (pH: 8.0) were used. For such similar measurements, HEPES is often used; however, it has a sulfo group (SO₃⁻) and hence may react with DTNB. For this reason, Tricine was also used. As an oxidizing reagent, 0.1 mM DTNB was used. Reaction solutions containing SDS were adjusted to a concentration of 0.02 % SDS. In this way, under conditions of three buffer solutions and with or without SDS, six reaction reagents in total were prepared.

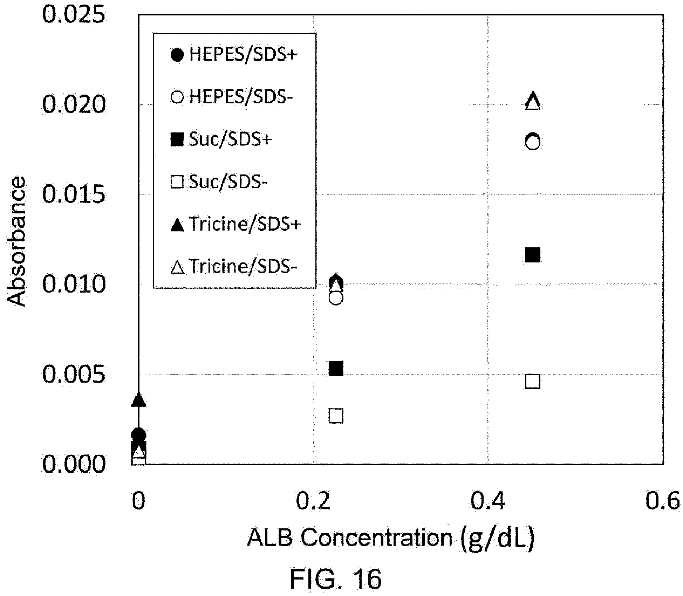

The samples and the reaction reagents were mixed together, subsequently heated at 37°C, and, after a lapse of 5 minutes, measured in terms of absorbance at 412 nm. DTNB reacts with the reduced cysteine residue of albumin and the resultant compound has a maximum absorption wavelength at 412 nm. Fig. 16 illustrates absorbances for the three buffer solutions, the succinic acid solution, the HEPES solution (HEPES, circles), and the Tricine solution (Tricine, triangles), with SDS (SDS+, black and filled) or without SDS (SDS-, white and open).

For the succinic acid solutions having a pH of 5.4 (Suc, squares), differences in absorbance were observed between with SDS (SDS+, black and filled) and without SDS (SDS-, white and open). Specifically, it was demonstrated that, in weakly acidic conditions, the oxidation reaction does not sufficiently proceed without SDS.

On the other hand, for the HEPES solutions (HEPES, circles) and the Tricine solutions (Tricine, triangles) having pH = 8.0, substantially no difference was observed in absorbance between with SDS (SDS+, black and filled) and without SDS (SDS-, white and open). Specifically, it was demonstrated that, in weakly alkaline conditions, oxidation sufficiently proceeds irrespective of with or without SDS. Without SDS, deformation of high-order structures of proteins can be avoided and, for example, the decrease in the activity of protease can be avoided.

### <Apparatus Configuration Example 1>

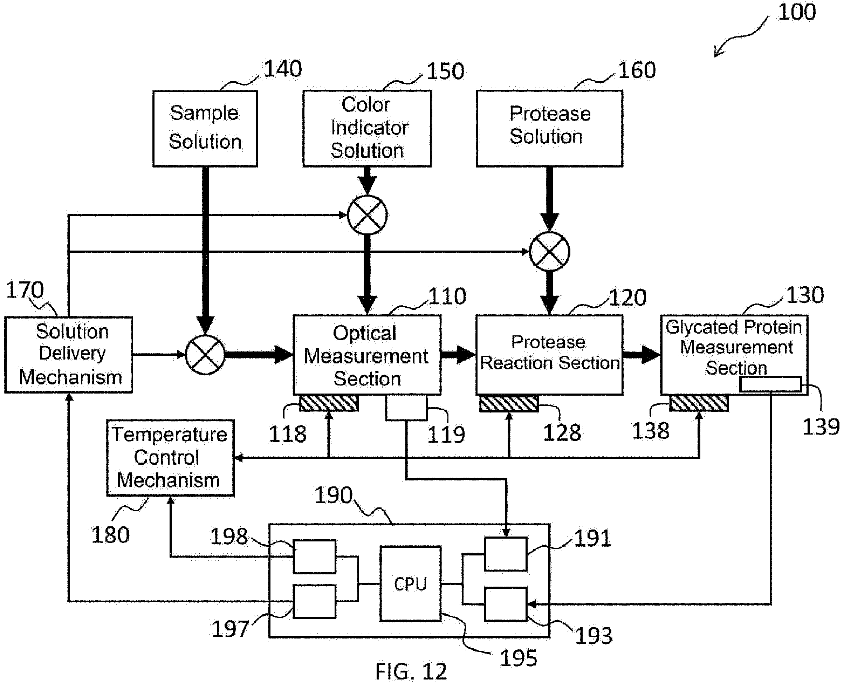

Fig. 12 schematically illustrates the configuration of an apparatus 100 according to an embodiment. The apparatus 100 includes an optical measurement section 110, a protease reaction section 120, and a glycated protein measurement section 130. The sample solution is introduced through an introduction unit 140 thereof to the optical measurement section 110 and sequentially caused to flow to the protease reaction section 120 and the glycated protein measurement section 130. A color indicator tank 150 is connected to the optical measurement section 110 and configured to provide a color indicator to the optical measurement section 110. A protease solution tank 160 is connected to the protease reaction section 120 and configured to provide a protease solution to the protease reaction section 120.

The introduction and delivering of the solutions are controlled by a solution flow mechanism 170. The solution delivery mechanism 170 can control the timing and amount of introduction of the sample solution into the optical measurement section 110. Similarly, the solution delivery mechanism 170 can control the timing and amount of introduction of the color indicator solution from the color indicator solution tank 150 to the optical measurement section 110 and the timing and amount of introduction of the protease solution from the protease solution tank 160 to the protease reaction section 120.

The solution delivery mechanism 170 may include, for example, a pump. In a case where the optical measurement section 110, the protease reaction section 120, and the glycated protein measurement section 130 are a closed channel, a positive pressure can be applied to an upstream region to control transfer of the solution. The solution delivery mechanism 170 may be press mechanisms attached to the solution tanks. When the solution tanks are containers whose shapes can be changed, the shapes of the containers can be changed, to thereby send out predetermined amounts of the solutions to the outside.

Temperature control units 118, 128, and 138 including a temperature sensor and a heater (not shown) are respectively disposed in the optical measurement section 110, the protease reaction section 120, and the glycated protein measurement section 130. The temperature control units 118, 128, and 138 are connected to a temperature control mechanism (driver) 180, which can receive measured temperature data from the temperature sensors and send instructions of turning on or off of heating and electric power to the heaters.

An optical measurement unit 119 including a light-emitting element and a light-receiving element (not shown) is disposed in the optical measurement section 110. Light emitted from the light-emitting element passes through the solution within the optical measurement section 110 and the transmitted light is detected by the light-receiving element. The optical measurement unit 119 can output the absorbance data at this time.

A sensor 139 is disposed in the glycated protein measurement section 130. The sensor 139 is disposed within the glycated protein measurement section 130 and can come into direct contact with the internal solution. This sensor 139 is configured to measure glycated protein concentration.

The apparatus 100 further includes a computer system 190. The computer system 190 at least includes a central processing unit (CPU) 195, which is configured to perform necessary mathematical operations. The computer system 190 includes communication interfaces 197 and 198 respectively for the solution delivery mechanism 170 and the temperature control mechanism 180. The computer system 190 includes communication interfaces 191 and 193 respectively for the optical measurement unit 119 and the sensor 139.

The CPU 195 transmits, via the solution-delivery-mechanism communication interface 197, to the solution delivery mechanism 170, necessary data such as necessary operation items (the solutions to be introduced and the introduction-target components), the timings, and the amounts. The solution delivery mechanism 170 executes necessary operations on the basis of the received data. The solution delivery mechanism 170 can transmit, for example, data of completion of execution of operations or data of execution failures to the communication interface 197.

The CPU 195 transmits, via the temperature-control-mechanism communication interface 198, to the temperature control mechanism 180, necessary data such as necessary operation items (such as temperature measurements and temperatures necessary for reactions), the timings, the temperatures, and the times. The temperature control mechanism 180 executes, on the basis of the received data, necessary operations. The temperature control mechanism 180 can transmit, for example, data of completion of execution of operations or data of execution failures to the communication interface 198.

The optical-measurement-unit communication interface 191 receives data related to absorbance from the optical measurement unit 119 disposed in the optical measurement section 110. The glycated-protein-sensor communication interface 193 receives data related to glycated protein concentration from the sensor 139 disposed in the glycated protein measurement section 130.

The optical-measurement-unit communication interface 191 instructs the optical measurement unit 119 disposed in the optical measurement section 110, to, for example, start or end the absorbance measurement, and receives data related to the measured protein concentration. The glycated-protein-sensor communication interface 193 instructs the sensor 139 disposed in the glycated protein measurement section 130, to, for example, start or end the current measurement, and receives data related to the measured glycated protein concentration.

The CPU 195 is configured to receive, via the communication interfaces 191 and 193, data related to absorbance and data related to glycated protein, to perform predetermined conversion, and to calculate the degree of glycation.

### <Apparatus Configuration Example 2>

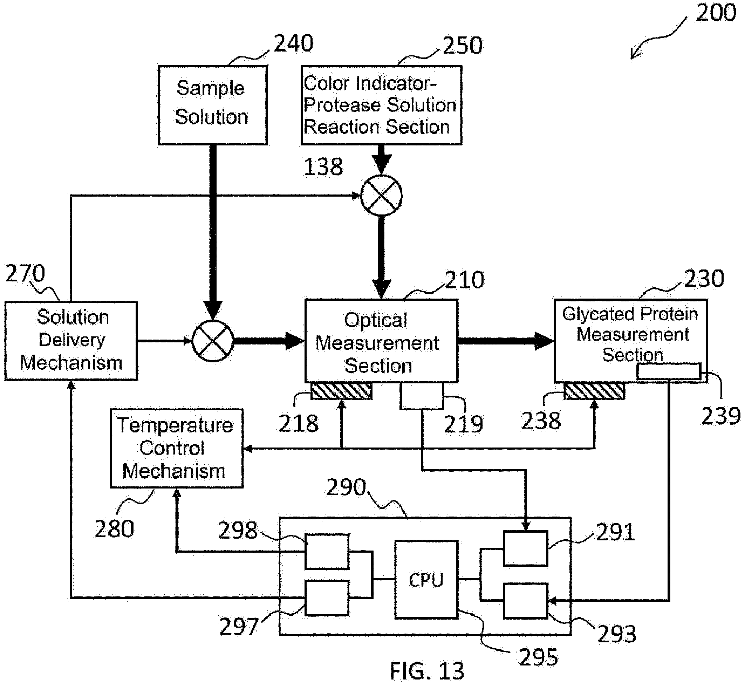

Fig. 13 schematically illustrates the configuration of an apparatus 200 according to an embodiment. The apparatus 200 includes an optical measurement section 210 and a glycated protein measurement section 230. The sample solution is introduced through an introduction unit 240 thereof into the optical measurement section 210 and caused to flow to the glycated protein measurement section 230. A measurement solution (color indicator-protease solution) tank 250 is connected to the optical measurement section 210 and configured to provide, to the optical measurement section 210, a mixture solution of a color indicator and protease.

The introduction and delivering of the solutions are controlled by a solution delivery mechanism 270.

Temperature control units 218 and 238 including a temperature sensor and a heater (not shown) are respectively disposed in the optical measurement section 210 and the glycated protein measurement section 230.

An optical measurement unit 219 is disposed in the optical measurement section 210 and can measure absorbance and output data related to the measured absorbance.

A sensor 239 is disposed in the glycated protein measurement section 230 and configured to measure glycated protein concentration and output data related to the measured glycated protein.

The apparatus 200 further includes a computer system 290. The computer system 290 at least includes a central processing unit (CPU) 295, which is configured to perform necessary mathematical operations. The computer system 290 can communicate, via communication interfaces 297 and 298, with the solution delivery mechanism 270 and the temperature control mechanism 280. The computer system 290 can communicate, via communication interfaces 291 and 293, with the optical measurement unit 219 and the sensor 239.

The CPU 295 is configured to receive, via the communication interfaces 291 and 293, data related to absorbance and data related to glycated protein, to perform predetermined conversion, and to calculate the degree of glycation.

### <Apparatus Configuration Example 3>

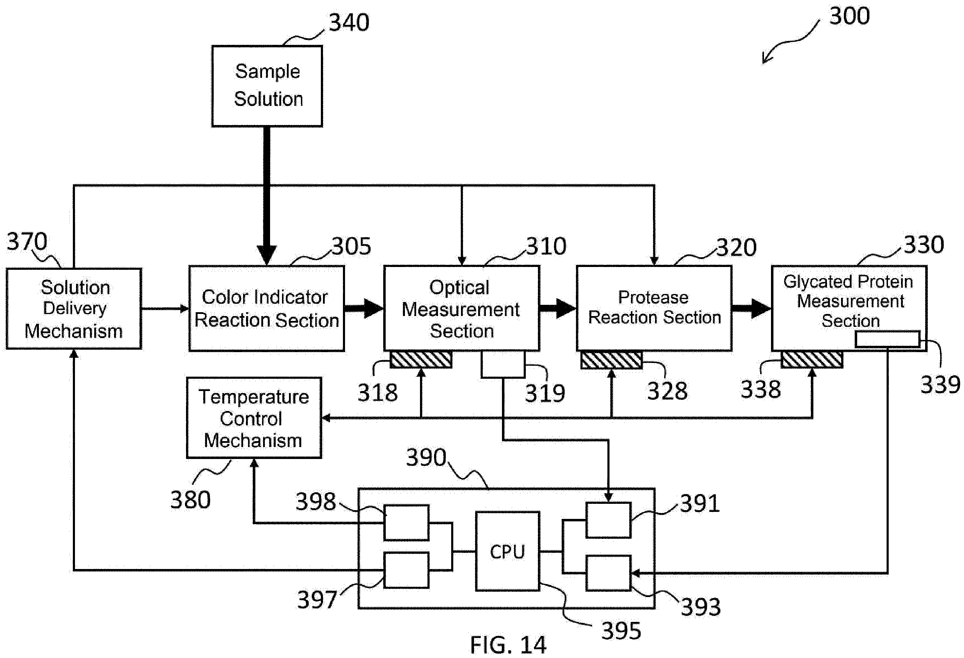

Fig. 14 schematically illustrates the configuration of an apparatus 300 according to an embodiment. The apparatus 300 includes a color indicator reaction section 305, an optical measurement section 310, a protease reaction section 320, and a glycated protein measurement section 330. The sample solution is introduced through an introduction unit 340 thereof to the color indicator reaction section 305 and sequentially caused to flow to the optical measurement section 310, the protease reaction section 320, and the glycated protein measurement section 330.

The color indicator reaction section 305 contains a color indicator in advance. The solution introduced into the color indicator reaction section 305 is mixed with and reacts with the color indicator. The protease reaction section 320 contains protease in advance. The solution introduced into the protease reaction section 320 comes into contact with and reacts with the protease.

The introduction and delivering of the solutions are controlled by a solution delivery mechanism 370.

Temperature control units 318, 328, and 338 including a temperature sensor and a heater (not shown) are respectively disposed in the optical measurement section 310, the protease reaction section 320, and the glycated protein measurement section 330.

An optical measurement unit 319 is disposed in the optical measurement section 310 and can measure absorbance and output data related to the measured absorbance.

A sensor 339 is disposed in the glycated protein measurement section 330 and configured to measure glycated protein concentration and output data related to the measured glycated protein.

The apparatus 300 further includes a computer system 390. The computer system 390 at least includes a central processing unit (CPU) 395, which is configured to perform necessary mathematical operations. The computer system 390 can communicate, via communication interfaces 397 and 398, the solution delivery mechanism 370 and the temperature control mechanism 380. The computer system 390 can communicate, via communication interfaces 391 and 393, the optical measurement unit 319 and the sensor 339.

The CPU 395 is configured to receive, via the communication interfaces 391 and 393, data related to absorbance and data related to glycated protein, to perform predetermined conversion, and to calculate the degree of glycation.

### <Apparatus Configuration Example 4>

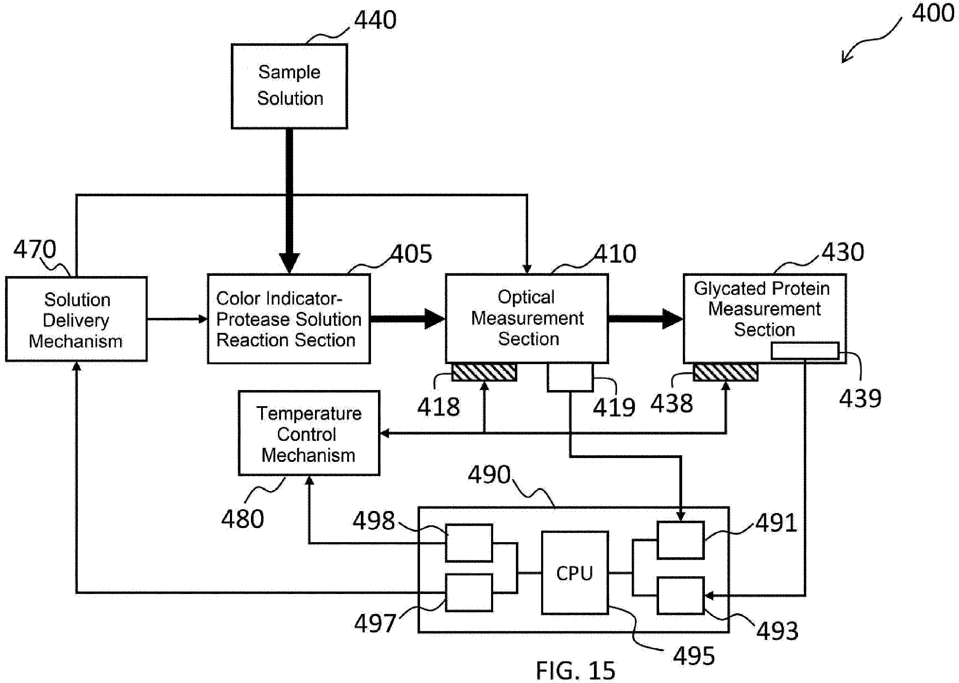

Fig. 15 schematically illustrates the configuration of an apparatus 400 according to an embodiment. The apparatus 400 includes a color indicator-protease solution reaction section 405, an optical measurement section 410, and a glycated protein measurement section 430. The sample solution is introduced through an introduction unit 440 thereof to the color indicator reaction section 405, and sequentially caused to flow to the optical measurement section 410 and the glycated protein measurement section 430.

The color indicator-protease solution reaction section 405 contains a color indicator and protease in advance. The solution having been introduced into the color indicator-protease solution reaction section 405 is mixed with and reacts with the color indicator and comes into contact with and reacts with the protease.

The introduction and delivering of the solutions are controlled by a solution delivery mechanism 470.

Temperature control units 418 and 438 including a temperature sensor and a heater (not shown) are disposed in the optical measurement section 410 and the glycated protein measurement section 430.

An optical measurement unit 419 is disposed in the optical measurement section 410 and can measure absorbance and output data related to the measured absorbance.

A sensor 439 is disposed in the glycated protein measurement section 430 and configured to measure glycated protein concentration and output data related to the measured glycated protein.

The apparatus 400 further includes a computer system 490. The computer system 490 at least includes a central processing unit (CPU) 495, which is configured to perform necessary mathematical operations. The computer system 490 can communicate, via communication interfaces 497 and 498, with the solution delivery mechanism 470 and the temperature control mechanism 480. The computer system 490 can communicate, via communication interfaces 491 and 493, with the optical measurement unit 419 and the sensor 439.

The CPU 495 is configured to receive, via the communication interfaces 491 and 493, data related to absorbance and data related to glycated protein, to perform predetermined conversion, and to calculate the degree of glycation.

The apparatus configuration is not limited to the above-described examples. In some embodiments, the apparatus or system may be an automatic dispensing apparatus or system (may also be simply referred to as automatic dispensing apparatus). The automatic dispensing apparatus may include a well for containing a reagent or a mixture solution of at least two reagents. In some embodiments, a cartridge including a plurality of wells (microplate) may be provided. The apparatus or cartridge may include an electrochemical sensor or may have a configuration to which an electrochemical sensor is attachable. Pipetting may be performed to quantify, transfer from a well to another well, or mix the samples and solutions. The automatic dispensing apparatus may include a heater and the heater may control the temperature of the well or the solution within the well. The automatic dispensing apparatus may include an optical measurement system.

In some embodiments, the apparatus may include a waste fluid tank. The solution having passed through the optical measurement section, the glycated protein measurement section, and the like may be collected into the waste fluid tank. This can reduce the risk that solutions containing body fluids leak to the outside.

The present disclosure also provides the following embodiments.
A001 A method for evaluating a degree of glycation of a protein, the method including:
   providing a solution potentially containing a target protein;
   bringing the solution into contact with a color indicator;
   bringing the solution into contact with protease;
   using a first reaction between the color indicator and the target protein to determine a concentration of the target protein in the solution;
   using a second reaction between the protease and the target protein to determine a concentration of the target protein having been glycated; and
   determining, based on the concentration of the target protein and the concentration of the target protein having been glycated, a degree of glycation of the target protein.
A011 The method according to Embodiment A001,
   wherein the bringing the solution into contact with the protease includes adding the protease to a color-indicator mixture solution generated by bringing the solution into contact with the color indicator.
A012 The method according to Embodiment A001,
   wherein the bringing the solution into contact with the color indicator and the bringing the solution into contact with the protease are performed substantially at the same time.
A012b The method according to Embodiment A001,
   including mixing substantially at the same time the color indicator and the protease with the solution.
A013 The method according to Embodiment A001,
   wherein the bringing the solution into contact with the color indicator includes adding, to a protease mixture solution generated by bringing the solution into contact with the protease, the color indicator.
A021 The method according to any one of Embodiments A001 to A013,
   wherein measuring the first reaction between the color indicator and the target protein includes measuring an absorbance of the color indicator.
A022 The method according to Embodiment A021,
   wherein the measuring the absorbancy of the color indicator includes measuring a change in the absorbance with time.
A023 The method according to Embodiment A022,
   wherein the measuring the change in the absorbance with time includes measuring the absorbance at at least two (or a plurality of) time points.
A024 The method according to Embodiment A022 or A023,
   wherein, based on the change in the absorbance with time, the concentration of the target protein having been glycated is determined.
A031 The method according to any one of Embodiments A001 to A024, wherein the target protein is albumin.
A032 The method according to Embodiment A031,
   wherein the using the second reaction between the protease and the albumin to determine a concentration of glycated albumin includes:
   using the protease to degrade the albumin to generate peptides and causing the generated peptides to react with ketoamine oxidase to generate hydrogen peroxide;
   measuring a concentration of the generated hydrogen peroxide; and
   determining, based on the concentration of the hydrogen peroxide, a concentration of the glycated albumin.
A033 The method according to Embodiment A032,
   wherein the measuring the concentration of the hydrogen peroxide includes using a hydrogen peroxide electrode.
A034 The method according to Embodiment A033,
   wherein the measuring the concentration of the hydrogen peroxide includes bringing the hydrogen peroxide having passed through an ion-exchange resin, into contact with the hydrogen peroxide electrode.
A041 The method according to any one of Embodiments A001 to A034,
   wherein the color indicator is a pH-indicator.
A042 The method according to any one of Embodiments A031 to A034,
   wherein the color indicator is bromocresol purple (BCP) or bromocresol green (BCG).
A051 The method according to Embodiment A042,
   wherein the color indicator is bromocresol purple (BCP) or bromocresol green (BCG),
   the method further including adjusting, during the first reaction, a pH of the solution to a range of 4.5 to 8.5 and adjusting, during the second reaction, a pH of the solution to a range of 6.0 to 9.0.
A052 The method according to any one of Embodiments A001 to A042,
   wherein, when the first reaction between the color indicator and the target protein and the second reaction between the color indicator and the target protein are performed (at least partially) at the same time, a pH of the solution is adjusted to a range suitable for both of the first reaction and the second reaction.
A053 The method according to Embodiment A052,
   wherein the color indicator is bromocresol purple (BCP) or bromocresol green (BCG), and
   the pH is adjusted to a range of 7.0 to 8.5.
A054 The method according to Embodiment A051 or A053,
   wherein the pH is adjusted to a range of 7.0 to 8.5, and
   during the first reaction, a mixture solution of the solution does not contain (contains none of) a protein denaturing agent (SDS).
A055 The method according to any one of Embodiments A032 to A054, wherein the color indicator is bromocresol purple (BCP) or bromocresol green (BCG),
   the method further including adjusting, during the first reaction, a temperature of the solution to a range of 20°C to 70°C, and adjusting, during the second reaction, a temperature of the solution to a range of 25°C to 70°C.
A056 The method according to any one of Embodiments A001 to A054,
   wherein, when the first reaction between the color indicator and the target protein and the second reaction between the color indicator and the target protein are performed (at least partially) at the same time, a temperature of the solution is adjusted to a range suitable for both of the first reaction and the second reaction.
A057 The method according to Embodiment A056,
   wherein the temperature is adjusted to a range of 25°C to 70°C.
A058 The method according to any one of Embodiments A032 to A034,
   the method further including adjusting a temperature of the ketoamine oxidase to a range of 25°C to 75°C.
A061 The method according to any one of Embodiments A032 to A058,
   wherein the ketoamine oxidase is an oxidase that acts on an amino acid or peptide in which an α-amino group is glycated (group I ketoamine oxidase) and the measuring the concentration of the generated hydrogen peroxide includes using a standard solution containing at least one of fructosyl valine, fructosyl glycine, or fructosyl valyl histidine to perform measurement calibration.
A062 The method according to any one of Embodiments A032 to A058,
   wherein the ketoamine oxidase is an oxidase that acts on an amino acid or peptide in which an ε-amino group is glycated (group II ketoamine oxidase) and the measuring the concentration of the generated hydrogen peroxide includes using a standard solution containing fructosyl lysine to perform measurement calibration.
A063 The method according to any one of Embodiments A032 to A058,
   wherein the ketoamine oxidase is an oxidase that acts on an amino acid or peptide in which an α-amino group and/or an ε-amino group is glycated (group III ketoamine oxidase), and
   the measuring the concentration of the generated hydrogen peroxide includes using a standard solution containing at least one of fructosyl lysine, fructosyl valine, fructosyl glycine, or fructosyl valyl histidine to perform measurement calibration.
B01 A program for executing the method according to any one of Embodiments A001 to A063 by a computer system.
B11 A storage medium storing the program according to Embodiment B01.
C01 An apparatus for evaluating a degree of glycation of a protein, the apparatus including:
   an introduction unit (or an introduction port) for a body fluid;
   a mechanism configured to mix together the body fluid and a color indicator solution;
   a protein concentration measurement section including an optical measurement unit configured to measure a protein concentration in a mixture solution of the body fluid and the color indicator solution;
   a mechanism configured to mix together the body fluid and a protease solution;
   a glycated protein measurement section including an electrode unit (sensor) configured to measure a glycated protein concentration in a mixture solution of the body fluid and the protease; and
   a processing section configured to communicate with the albumin concentration measurement section and the glycated protein measurement section.
C02 The apparatus according to Embodiment C01,
   wherein the mixing together the body fluid and the protease solution includes mixing the mixture solution of the body fluid and the color indicator solution with the protease solution.
C11 The apparatus according to Embodiment C01 or C02,
   wherein the protein concentration measurement section and the glycated protein measurement section are in fluid communication in series.
C12 The apparatus according to Embodiment C11,
   wherein the introduction unit for the body fluid, the protein concentration measurement section, and the glycated protein measurement section are in fluid communication in series in this order.
C13 The apparatus according to Embodiment C01 or C02,
   wherein the protein concentration measurement section and the glycated protein measurement section are disposed in parallel and connected to the introduction unit.
C21 The apparatus according to any one of Embodiments C01 to C13,
   further including a waste fluid section (tank) in fluid communication with a downstream region of the protein concentration measurement section and the glycated protein measurement section.
C31 The apparatus according to any one of Embodiments C01 to C21,
   wherein the optical measurement unit includes an absorptiometer.
C41 The apparatus according to any one of Embodiments C01 to C31,
   wherein the electrode unit (sensor) configured to measure the glycated protein concentration includes ketoamine oxidase and a hydrogen peroxide electrode.
C42 The apparatus according to Embodiment C41,
   wherein the sensor further includes an ion exchange membrane covering the hydrogen peroxide electrode.
C43 The apparatus according to Embodiment C41 or C42,
   further including an amperometer configured to measure a current generated in the hydrogen peroxide electrode.
C51 The apparatus according to any one of Embodiments C01 to C043, further including a color indicator solution tank containing the BCP solution and a protease solution tank containing the protease solution.
C52 The apparatus according to any one of Embodiments C01 to C043, further including a treatment solution tank containing a mixture solution of the color indicator solution and the protease solution.
C61 The apparatus according to any one of Embodiments C01 to C52,
   wherein the protein is albumin.
C62 The apparatus according to Embodiment C61,
   wherein the color indicator is BCP or BCG.
C71 The apparatus according to any one of C01 to C62,
   wherein the apparatus is or includes an automatic dispensing apparatus or device.

While several embodiments and examples of the present disclosure have been described above, these embodiments and examples are used for exemplarily explanations of the present disclosure. For example, each of the embodiments described above has been described in detail in order to explain the present disclosure in an easy-to-understand manner, and dimensions, configurations, materials, and circuits may be additionally changed as necessary. Embodiments in which one or more of the above-mentioned features of the present disclosure are arbitrarily combined are also included in the scope of the present disclosure. It is intended that the appended claims cover numerous modifications to the embodiments without departing from the spirit and scope of the present disclosure. Accordingly, the embodiments and examples disclosed herein are presented for purposes of illustration and should not be construed as limiting the scope of the present disclosure.

## Claims

1. A method for evaluating a degree of glycation of a protein, the method comprising:
providing a solution potentially containing a target protein;
bringing the solution into contact with a color indicator;
bringing the solution into contact with protease;
using a first reaction between the color indicator and the target protein to determine a concentration of the target protein in the solution;
using a second reaction between the protease and the target protein to determine a concentration of the target protein having been glycated; and
determining a degree of glycation of the target protein, based on the concentration of the target protein and the concentration of the target protein having been glycated.

2. The method according to claim 1,
wherein the bringing the solution into contact with the protease includes adding the protease to a color-indicator mixture solution generated by bringing the solution into contact with the color indicator.

3. The method according to claim 1,
wherein the bringing the solution into contact with the color indicator and the bringing the solution into contact with the protease are performed substantially at the same time.

4. The method according to any one of claims 1 to 3,
wherein measuring the first reaction between the color indicator and the target protein includes measuring an absorbance of the color indicator.

5. The method according to claim 4,
wherein the measuring the absorbancy of the color indicator includes measuring a change in the absorbance with time.

6. The method according to any one of claims 1 to 5,
wherein the target protein is albumin.

7. The method according to claim 6,
wherein the using the second reaction between the protease and the albumin to determine a concentration of glycated albumin includes:
using the protease to degrade the albumin to generate peptides and causing the generated peptides to react with ketoamine oxidase to generate hydrogen peroxide;
measuring a concentration of the generated hydrogen peroxide; and
determining, based on the concentration of the hydrogen peroxide, the concentration of the glycated albumin.

8. The method according to claim 7,
wherein the measuring the concentration of the hydrogen peroxide includes using a hydrogen peroxide electrode.

9. The method according to claim 8,
wherein the measuring the concentration of the hydrogen peroxide includes bringing the hydrogen peroxide having passed through an ion-exchange resin, into contact with the hydrogen peroxide electrode.

10. The method according to any one of claims 1 to 9,
wherein the color indicator is a pH-indicator.

11. The method according to any one of claims 6 to 9,
wherein the color indicator is bromocresol purple (BCP) or bromocresol green (BCG).

12. The method according to any one of claims 7 to 9,
wherein the color indicator is bromocresol purple (BCP) or bromocresol green (BCG),
the method further comprising adjusting, during the first reaction, a pH of the solution to a range of 4.5 to 8.5 and adjusting, during the second reaction, a pH of the solution to a range of 6.0 to 9.0.

13. The method according to any one of claims 1 to 11,
wherein, when the first reaction between the color indicator and the target protein and the second reaction between the color indicator and the target protein are performed at the same time, a pH of the solution is adjusted to a range suitable for both of the first reaction and the second reaction.

14. The method according to claim 13,
wherein the color indicator is bromocresol purple (BCP) or bromocresol green (BCG), and
the pH is adjusted to a range of 7.0 to 8.5.

15. The method according to claim 12 or 14,
wherein the pH is adjusted to a range of 7.0 to 8.5, and
in the first reaction, a mixture solution of the solution does not contain a protein denaturing agent.

16. The method according to any one of claims 7 to 9,
wherein the color indicator is bromocresol purple (BCP) or bromocresol green (BCG),
the method further comprising, adjusting, during the first reaction, a temperature of the solution to a range of 20°C to 70°C, and adjusting, during the second reaction, a temperature of the solution to a range of 25°C to 70°C.

17. The method according to any one of claims 1 to 16,
wherein, when the first reaction between the color indicator and the target protein and the second reaction between the color indicator and the target protein are performed at least partially at the same time, a temperature of the solution is adjusted to a range suitable for both of the first reaction and the second reaction.

18. The method according to claim 17,
wherein the temperature is adjusted to a range of 25°C to 70°C.

19. The method according to any one of claims 7 to 9,
the method further comprising adjusting a temperature of the ketoamine oxidase to a range of 25°C to 75°C.

20. The method according to any one of claims 7 to 19,
wherein the ketoamine oxidase is an oxidase that acts on an amino acid or peptide in which an ε-amino group is glycated (group II ketoamine oxidase) and the measuring the concentration of the generated hydrogen peroxide includes using a standard solution containing fructosyllysine to perform measurement calibration.
